# EUROPEAN PATENT APPLICATION

(11) **EP 4 800 649 A1**
(43) Date of publication of application: **02.09.2026**
(21) Application number: 26160955.6
(22) Date of filing: 26.02.2026
(51) Int. Cl.: G06T 7/62

(54) **DIMENSION EVALUATION SYSTEM AND METHOD THEREOF**

(30) Priority: 26.02.2025 TW 114107207
(71) Applicant: ASUSTek Computer Inc., Peitou, Taipei-City 112 (TW)
(72) Inventor: WANG, Chia-Yu, 112 Taipei City (TW); CHEN, Yu-Hsin, 112 Taipei City (TW); SHEN, Yu-Jie, 112 Taipei City (TW)
(74) Representative: Viering, Jentschura & Partner mbB Patent- und Rechtsanwälte

(57) **Abstract**

A dimension evaluation system (10) includes a computing device (12) and a display device (14). An anomaly detection model (16), a three-dimensional feature prediction model (18), and a feature tracking prediction model (20) are built in the computing device (12). The anomaly detection model (16) detects an anomalous feature (28) on an instant image (26), to mark a selection box (30), and obtains location information. The three-dimensional feature prediction model (18) calculates a dimension of the anomalous feature (28) based on the instant image (26) and the location information. The feature tracking prediction model (20) performs mathematical statistics on all dimensions of the anomalous feature (28) when it is determined that anomalous features (28) detected in two consecutive pictures of the instant image (26) are the same, and generates a mathematical statistics result (32) of the dimension of the anomalous feature (28) when the computing device (12) receives a picture static signal. The display device (14) displays an instant image (26), a selection box (30), and a mathematical statistics result (32).

## Description

### BACKGROUND OF THE INVENTION

### Field of the Invention

The disclosure relates to a dimension evaluation system for obtaining a mathematical statistics result of a dimension of an anomalous feature and a method thereof.

### Description of the Related Art

An endoscopy instrument is a device for inspecting an organ or a structure in a human body by using an endoscope. The device enters the human body through various channels and observes conditions inside the human body, to determine whether there is any lesion. A common colonoscope is used as an example, and the colonoscope uses a soft-fiber endoscope that enters the large intestine for direct observation and inspection. Generally, a colonoscopy instrument includes a special elongated flexible hose and a small camera at a head end of the hose. The colonoscopy instrument enters a location of the intestine from the anus, and observes along a wall of the hose whether there is a lesion such as a polyp or a tumor. During colonoscopy, the colonoscopy instrument is connected to a monitor, to display a photographed instant image of an internal structure of the intestine on the monitor, to allow a doctor to check or diagnose a health status inside a large intestine of a subject.

During colonoscopy, when a polyp is photographed by the endoscope, a dimension of the polyp is usually calculated through a single frame of image. However, when the dimension of the polyp is measured, due to different image capture angles of the polyp or slight differences in images, errors in measurement data may be generated. Therefore, a problem that results of measured dimensions of the same polyp in different frames of images are inconsistent occurs, which is prone to disputes.

### BRIEF SUMMARY OF THE INVENTION

The disclosure provides a dimension evaluation system. The dimension evaluation system is adapted for being electrically connected to a detecting instrument. The detecting instrument inspects a target object and generates an instant image. The dimension evaluation system includes a computing device and a display device. The computing device is connected to the detecting instrument through a signal, and is built with an anomaly detection model, a three-dimensional feature prediction model, and a feature tracking prediction model. The computing device receives the instant image, the anomaly detection model detects an anomalous feature on the instant image, to mark a selection box around the anomalous feature, and to obtain location information. The three-dimensional feature prediction model calculates a dimension of the anomalous feature based on the instant image and the location information. The feature tracking prediction model performs mathematical statistics on all dimensions of the same anomalous feature when it is determined that an anomalous feature detected in each current picture in the instant image is the same as an anomalous feature detected in a previous picture in the instant image, and generates a mathematical statistics result of a dimension corresponding to the anomalous feature when the computing device receives a picture static signal. The display device is electrically connected to the computing device. The display device is configured to display the instant image, the selection box, and the mathematical statistics result.

The disclosure further provides a dimension evaluation method, applicable to an instant image generated by a detecting instrument by inspecting a target object. The dimension evaluation method includes: receiving the instant image and detecting an anomalous feature on the instant image, to mark a selection box around the anomalous feature and to obtain location information; calculating a dimension of the anomalous feature based on the instant image and the location information; performing mathematical statistics on all dimensions of a same anomalous feature when it is determined that an anomalous feature detected in each current picture in the instant image is the same as an anomalous feature detected in a previous picture in the instant image; generating a mathematical statistics result of a dimension corresponding to the anomalous feature when a picture static signal is received; and displaying the instant image, the selection box, and the mathematical statistics result.

In conclusion, in the dimension evaluation system and the method thereof in the disclosure, after the instant image is obtained, the mathematical statistics result of the dimension of the anomalous feature on the instant image may be evaluated through an built-in artificial intelligence (AI) model, and the instant image and a mathematical statistics result mark of the dimension of the anomalous feature on the instant image are directly displayed on the display device, to improve stability of a result of measuring the dimension of the anomalous feature. Therefore, the disclosure can effectively assist a doctor, to provide a stable and accurate anomalous feature dimension for the doctor to make more accurate diagnosis, and improve using experience of the doctor.

### BRIEF DESCRIPTION OF THE DRAWINGS

FIG. 1 is a schematic block diagram of a dimension evaluation system and a detecting instrument connected to the dimension evaluation system according to an embodiment of the disclosure;
FIG. 2 is a schematic flowchart of a dimension evaluation method according to an embodiment of the disclosure;
FIG. 3 is a schematic architectural diagram of displaying, on a timeline, an instant image marked with a selection box according to an embodiment of the disclosure;
FIG. 4 is a schematic architectural diagram of a dimension evaluation system and an instant image that is marked with a mathematical statistics result and that is displayed in the dimension evaluation system according to an embodiment of the disclosure; and
FIG. 5 is a schematic flowchart of performing tracking prediction by a dimension evaluation system using a feature tracking prediction model according to an embodiment of the disclosure.

### DETAILED DESCRIPTION OF THE EMBODIMENTS

Embodiments of the disclosure are described with reference to relevant drawings. In addition, some elements and structures are omitted in the drawings in the embodiments to clearly show the technical characteristics of the disclosure. In these drawings, the same reference numeral indicates the same or similar elements or circuits.

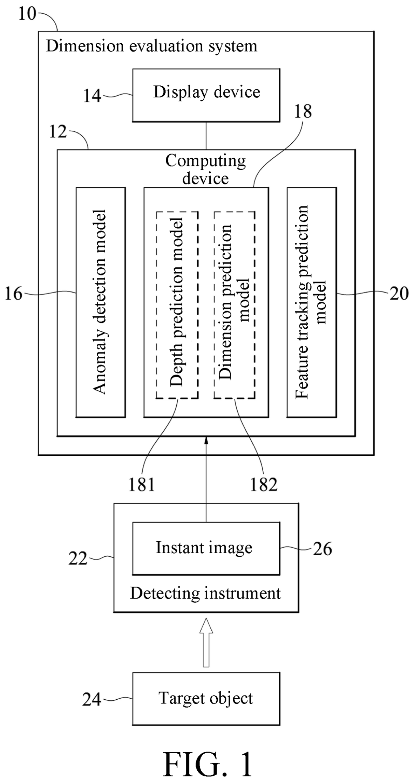

FIG. 1 is a schematic block diagram of a dimension evaluation system and a detecting instrument connected to the dimension evaluation system according to an embodiment of the disclosure. Referring to FIG. 1, a dimension evaluation system 10 is adapted for being electrically connected to a detecting instrument 22, and the detecting instrument 22 inspects and photographs a target object 24 and correspondingly generates an instant image 26, so as to transmit the instant image 26 to the dimension evaluation system 10. The dimension evaluation system 10 includes a computing device 12 and a display device 14. The computing device 12 is connected to a detecting instrument 22 through a signal. In an embodiment, the computing device 12 is connected to the detecting instrument 22 through a high-definition multimedia interface (HDMI), a universal serial bus (USB) interface, a serial digital interface (SDI), or the like. The computing device 12 is electrically connected to the display device 14. In an embodiment, the computing device 12 is connected to the display device 14 through a high-definition multimedia interface (HDMI), a display port (Display Port, DP) interface, a serial digital interface (SDI), or the like. An anomaly detection model 16, a three-dimensional feature prediction model 18, and a feature tracking prediction model 20 are built in the computing device 12. After the computing device 12 receives the instant image 26 from the detecting instrument 22, the computing device 12 performs computing processing on the instant image 26, to detect the anomalous feature by using the anomaly detection model 16, predict a dimension of the anomalous feature by using the three-dimensional feature prediction model 18, and track the anomalous feature by using the feature tracking prediction model 20, and performs mathematical statistics. The display device 14 is configured to display the instant image 26 processed by the computing device 12.

In an embodiment, the detecting instrument 22 is an endoscopic system, for example, a colonoscopy instrument. In this case, the target object 24 is the intestine. In an embodiment, the anomalous feature includes a hyperplastic tissue or a pathological tissue of the target object 24, for example, a polyp, a tumor, or another formation generated on the target object. In an embodiment, when the target object 24 is an intestine, the anomalous feature is a large intestine polyp.

In an embodiment, the computing device 12 is a computer host or another electronic device that can perform independent computing, and is used together with the display device 14. In another embodiment, in the disclosure, a notebook computer may be directly used to replace functions of the computing device 12 and the display device 14, so that the notebook computer is responsible for operation of the computing device 12 and the display device 14.

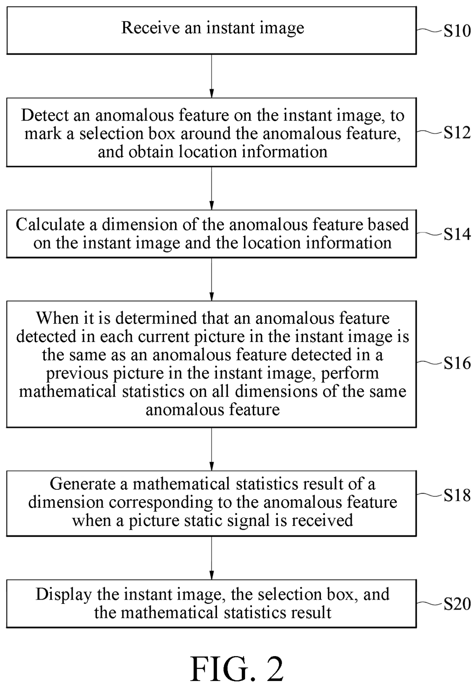

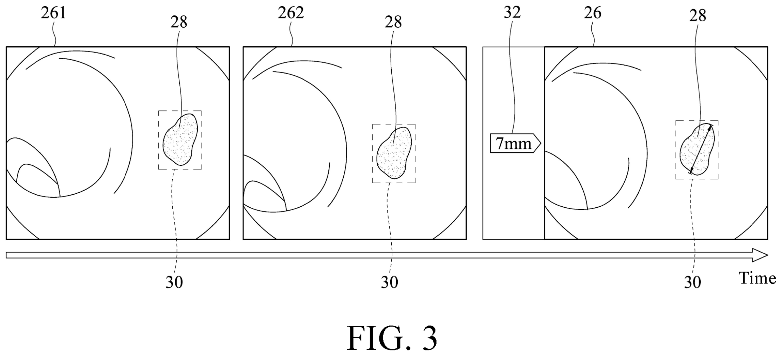

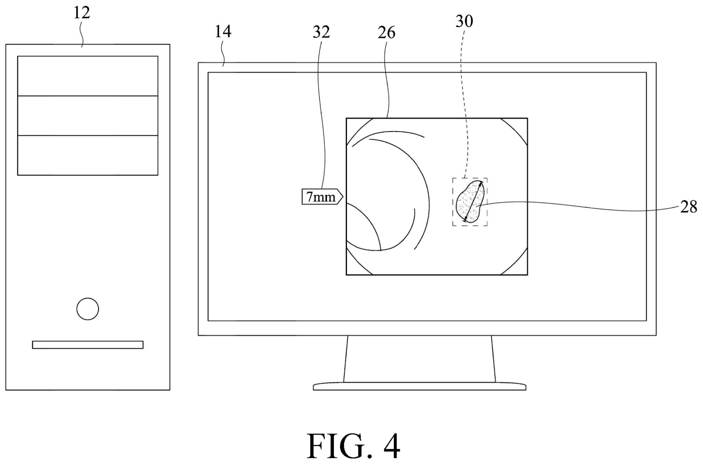

Continuing with the architecture shown in FIG. 1, procedures of steps of a dimension evaluation method performed by the dimension evaluation system 10 of the disclosure are described. Referring to both FIG. 1 and FIG. 2, after the detecting instrument 22 inspects the target object 24 and generates the instant image 26, as shown in step S10, the computing device 12 receives the instant image 26 from the detecting instrument 22. In this case, the computing device 12 transmits the instant image 26 to the display device 14, so that the instant image 26 is displayed on the display device 14. As shown in step S12, the computing device 12 detects an anomalous feature 28 on the instant image 26 by using the anomaly detection model 16, as shown in FIG. 3, to mark a selection box 30 around the anomalous feature 28, and obtains location information corresponding to the anomalous feature 28. As shown in step S14, the computing device 12 executes the three-dimensional feature prediction model 18, and the three-dimensional feature prediction model 18 calculates a dimension of the anomalous feature 28 based on the instant image 26 and the location information. As shown in step S16, the computing device 12 executes the feature tracking prediction model 20. When determining that an anomalous feature 28 detected in each current picture 262 in the instant image 26 is the same as an anomalous feature 28 detected in a previous picture 261 in the instant image 26, the feature tracking prediction model 20 performs mathematical statistics on all dimensions of the same anomalous feature 28. As shown in step S18, when the detecting instrument 22 is triggered to generate a picture static signal, the picture static signal is transmitted to the computing device 12. When the computing device 12 receives the picture static signal, the computing device 12 generates a mathematical statistics result 32 of all dimensions corresponding to the anomalous feature 28. Finally, as shown in step S20, referring to FIG. 3 and FIG. 4, the computing device 12 adds the mathematical statistics result 32 to the instant image 26 and performs outputting to the display device 14, so that the display device 14 displays the instant image 26, the selection box 30, and the mathematical statistics result 32.

In an embodiment, as shown in FIG. 1 and FIG. 3, the three-dimensional feature prediction model 18 further includes a depth prediction model 181 and a dimension prediction model 182. The depth prediction model 181 estimates a depth of the anomalous feature 28 based on the instant image 26 and the location information. The depth is a distance between a lens of the detecting instrument 22 and the anomalous feature 28 on the target object 24. After the depth of the anomalous feature 28 is obtained, the dimension prediction model 182 calculates a dimension corresponding to the anomalous feature 28 based on the location information and the depth.

In an embodiment, a mathematical statistics manner used by the computing device 12 is a mean (Mean), an arithmetic mean (arithmetic mean), a geometric mean (Geometric Mean), a harmonic mean (Harmonic Mean), a weighted mean (Weighted Mean), a trimmed mean (Trimmed Mean), a median (Median), a mode (Mode), percentiles (Percentiles), and the like.

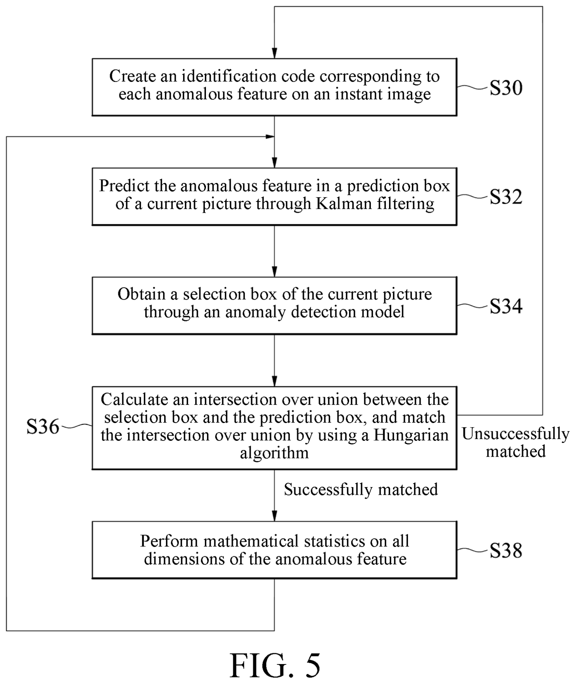

In an embodiment, referring to FIG. 1, FIG. 3, and FIG. 5, a step of performing tracking prediction by the computing device 12 using the feature tracking prediction model 20 further includes the following steps. First, as shown in step S30, an identification code corresponding to each anomalous feature 28 on the instant image 26 is created. As shown in step S32, the anomalous feature 28 is predicted in a prediction box (not shown in the figure) of the current picture 262 through Kalman filtering. As shown in step S34, a selection box 30 of the previous picture 261 is obtained through the anomaly detection model 16. As shown in step S36, an intersection over union (Intersection Over Union, IOU) between the selection box 30 and the prediction box is calculated, the intersection over union is matched by using a Hungarian algorithm, and when the intersection over union is successfully matched, it indicates that the selection box 30 and the prediction box are successfully matched. As shown in step S38, mathematical statistics are performed on all dimensions of the anomalous feature 28, and return to step S32 again. If the intersection over union is unsuccessfully matched, it indicates that the anomalous feature 28 is unsuccessfully matched or the selection box 30 is unsuccessfully matched, return to step S30 again to wait for detection of a new anomalous feature 28.

In an embodiment, the anomaly detection model 16, the three-dimensional feature prediction model 18 (including the depth prediction model 181 and the dimension prediction model 182), and the feature tracking prediction model 20 are respectively trained neural network models.

In conclusion, in the dimension evaluation system and the method thereof in the disclosure, after the instant image is obtained, the mathematical statistics result of the dimension of the anomalous feature on the instant image may be evaluated through an built-in artificial intelligence (AI) model, and the instant image and a mathematical statistics result mark of the dimension of the anomalous feature on the instant image are directly displayed on the display device, to improve stability of a result of measuring the dimension of the anomalous feature. Therefore, the disclosure can effectively assist a doctor, to provide a stable and accurate anomalous feature dimension for the doctor to make more accurate diagnosis, and improve using experience of the doctor.

The above-described embodiments are merely for describing the technical ideas and characteristics of the disclosure, and are intended to enable those skilled in the art to understand and hereby implement the content of the disclosure. However, the scope of claims of the disclosure is not limited thereto. In other words, equivalent changes or modifications made according to the spirit disclosed in the disclosure shall still fall into scope of the claims of the disclosure.

## Claims

1. A dimension evaluation system (10), adapted for being electrically connected to a detecting instrument (22), wherein the detecting instrument (22) inspects a target object (24) and generates an instant image (26), and the dimension evaluation system (10) comprises:
a computing device (12), connected to the detecting instrument (22) through a signal and built with an anomaly detection model (16), a three-dimensional feature prediction model (18), and a feature tracking prediction model (20), wherein the computing device (12) receives the instant image (26), the anomaly detection model (16) detects an anomalous feature (28) on the instant image (26), to mark a selection box (30) around the anomalous feature (28), and obtains location information, the three-dimensional feature prediction model (18) calculates a dimension of the anomalous feature (28) based on the instant image (26) and the location information, and when it is determined that an anomalous feature (28) detected in each current picture (262) in the instant image (26) is the same as an anomalous feature (28) detected in a previous picture (261) in the instant image (26), the feature tracking prediction model (20) performs mathematical statistics on all dimensions of the same anomalous feature (28), and generates a mathematical statistics result (32) of the dimension corresponding to the anomalous feature (28) when the computing device (12) receives a picture static signal; and
a display device (14), electrically connected to the computing device (12), wherein the display device (14) is configured to display the instant image (26), the selection box (30), and the mathematical statistics result (32).

2. The dimension evaluation system (10) according to claim 1, wherein the three-dimensional feature prediction model (18) further comprises a depth prediction model (181) and a dimension prediction model (182), the depth prediction model (181) estimates a depth of the anomalous feature (28) based on the instant image (26) and the location information, and the dimension prediction model (182) calculates the dimension of the anomalous feature (28) based on the location information and the depth.

3. The dimension evaluation system (10) according to claim 1, wherein the detecting instrument (22) is an endoscopic system.

4. The dimension evaluation system (10) according to claim 1, wherein the mathematical statistics is a mean (Mean), an arithmetic mean (arithmetic mean), a geometric mean (Geometric Mean), a harmonic mean (Harmonic Mean), a weighted mean (Weighted Mean), a trimmed mean (Trimmed Mean), a median (Median), a mode (Mode), or a percentile (Percentile).

5. The dimension evaluation system (10) according to claim 1, wherein a step of performing tracking prediction by the feature tracking prediction model (20) further comprises:
creating an identification code corresponding to each anomalous feature (28) on the instant image (26);
predicting the anomalous feature (28) in a prediction box of the current picture (262) through Kalman filtering;
obtaining a selection box (30) of the previous picture (261) through the anomaly detection model (16); and
calculating an intersection over union (Intersection Over Union, IOU) between the selection box (30) and the prediction box, matching the intersection over union by using a Hungarian algorithm, indicating that the selection box (30) and the prediction box are successfully matched when the intersection over union is successfully matched, and performing mathematical statistics on all the dimensions of the anomalous feature (28).

6. The dimension evaluation system (10) according to claim 5, wherein when the intersection over union is unsuccessfully matched, the anomalous feature (28) fails to be matched or the selection box (30) fails to be matched.

7. The dimension evaluation system (10) according to claim 1, wherein the anomalous feature (28) comprises a hyperplastic tissue or a pathological tissue of the target object (24).

8. The dimension evaluation system (10) according to claim 1, wherein the picture static signal is generated by the detecting instrument (22) being triggered.

9. A dimension evaluation method, applicable to an instant image (26) generated by a detecting instrument (22) by inspecting a target object (24), and the dimension evaluation method comprising:
receiving the instant image (26) and detecting an anomalous feature (28) on the instant image (26), to mark a selection box (30) around the anomalous feature (28) and to obtain location information;
calculating a dimension of the anomalous feature (28) based on the instant image (26) and the location information;
performing mathematical statistics on all dimensions of a same anomalous feature (28) when it is determined that an anomalous feature (28) detected in each current picture (262) in the instant image (26) is the same as an anomalous feature (28) detected in a previous picture (261) in the instant image (26);
generating a mathematical statistics result (32) of the dimension corresponding to the anomalous feature (28) when a picture static signal is received; and
displaying the instant image (26), the selection box (30), and the mathematical statistics result (32).

10. The dimension evaluation method according to claim 9, wherein the detecting instrument (22) is an endoscopic system.

11. The dimension evaluation method according to claim 9, wherein the anomalous feature (28) is detected by an anomaly detection model (16) and is marked with the selection box (30).

12. The dimension evaluation method according to claim 9, wherein the dimension is generated by a three-dimensional feature prediction model (18).

13. The dimension evaluation method according to claim 12, wherein the three-dimensional feature prediction model (18) further comprises a depth prediction model (181) and a dimension prediction model (182), the depth prediction model (181) estimates a depth of the anomalous feature (28) based on the instant image (26) and the location information, and the dimension prediction model (182) calculates the dimension of the anomalous feature (28) based on the location information and the depth.

14. The dimension evaluation method according to claim 9, wherein the mathematical statistics is a mean (Mean), an arithmetic mean (arithmetic mean), a geometric mean (Geometric Mean), a harmonic mean (Harmonic Mean), a weighted mean (Weighted Mean), a trimmed mean (Trimmed Mean), a median (Median), a mode (Mode), or a percentile (Percentile).

15. The dimension evaluation method according to claim 9, wherein a step of determining whether the anomalous features (28) are the same is performed by a feature tracking prediction model (20), and mathematical statistics are performed on all the dimensions of the same anomalous feature (28).

16. The dimension evaluation method according to claim 15, wherein a step of performing tracking prediction by the feature tracking prediction model (20) further comprises:
creating an identification code corresponding to each anomalous feature (28) on the instant image (26);
predicting the anomalous feature (28) in a prediction box of the current picture (262) through Kalman filtering;
obtaining a selection box (30) of the previous picture (261) through anomaly detection; and
calculating an intersection over union (Intersection Over Union, IOU) between the selection box (30) and the prediction box, matching the intersection over union by using a Hungarian algorithm, indicating that the selection box (30) and the prediction box are successfully matched when the intersection over union is successfully matched, and performing mathematical statistics on all the dimensions of the anomalous feature (28).

17. The dimension evaluation method according to claim 16, wherein when the intersection over union is unsuccessfully matched, the anomalous feature (28) fails to be matched or the selection box (30) fails to be matched.

18. The dimension evaluation method according to claim 9, wherein the anomalous feature (28) comprises a hyperplastic tissue or a pathological tissue of the target object (24).

19. The dimension evaluation method according to claim 9, wherein the picture static signal is generated by the detecting instrument (22) being triggered.
